# EUROPEAN PATENT SPECIFICATION

(11) **EP 1 675 833 B1**
(45) Date of publication and mention of the grant of the patent: **26.01.2011**
(21) Application number: 04791252.2
(22) Date of filing: 19.10.2004
(51) Int. Cl.: C07D 233/90, A61K 31/417, A61P 3/04

(54) **1H-IMIDAZOLE DERIVATIVES AS CANNABINOID RECEPTOR MODULATORS**
1H-IMIDAZOLDERIVATE ALS MODULATOREN DES CANNABINOIDREZEPTORS
DERIVES DE 1H-IMIDAZOLE COMME MODULATEURS DU RECEPTEUR DE CANNABINOIDES

(30) Priority: 20.10.2003 EP 03103867; 20.10.2003 US 512105 P
(43) Date of publication of application: 05.07.2006
(73) Proprietor: Abbott Healthcare Products B.V., 1381 CP Weesp (NL)
(72) Inventor: LANGE, Josephus H.M., NL-1381 CP Weesp (NL); KRUSE, Cornelis G., NL-1381 CP Weesp (NL); VAN STUIVENBERG, Herman H., NL-1381 CP Weesp (NL); KEIZER, Hiskias G., NL-1381 CP Weesp (NL)
(74) Representative: Aerts, Robert Jaap
(86) International application number: PCT/EP2004/052575
(87) International publication number: WO 2005/040130

(56) References cited:
- WO-A-03/027076
- WO-A-03/063781

## Description

The present invention relates to a group of 1H-imidazole derivatives which are modulators of cannabinoid receptors , to methods for the preparation of these compounds, to novel intermediates useful for the synthesis of said imidazole derivatives, to methods for the preparation of these intermediates, to pharmaceutical compositions containing one or more of these 1H-imidazole derivatives as active ingredient, as well as to the use of these pharmaceu tical compositions for the treatment of psychiatric and neurological disorders in which cannabinoid receptors are involved.

The invention also relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a disease or condition. More particularly, the invention relates to a new use for the treatment of a disease or condition disclosed herein or apparent to a person skilled in the art from the specification and general knowledge in the art. In embodiments of the invention specific compounds disclosed herein are used for the manufacture of a medicament.

1H-Imidazole derivatives as CB₁ receptor modulators are already known from WO 03/027076. However, the compounds described in WO 03/027076 are characterized by a relatively poor aqueous solubility. The goal of the present invention was to improve the aqueous solubility of 1H-imidazole derivatives whilst maintaining their cannabinoid receptor modulating activity. From the prior art it is not obv ious how this goal should be achieved.

Surprisingly, it has now been found that the introduction of a 5-aminomethyl moiety in the 1H-imidazole core results in an enhanced aqueous solubility. Moreover, the derivatives containing said 5-aminomethyl moiety retain their cannabinoid receptor modulating activity, such as receptor antagonism, receptor inverse agonism or receptor (partial) agonism.

The invention relates to compounds of the general formula (I) 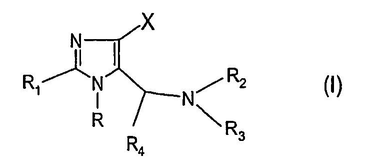 wherein:
- R and R₁ are the same or different and represent phenyl, thienyl, pyridinyl, pyrimidinyl, pyrazinyl, pyridazinyl or triazinyl, which groups may be substituted with 1, 2, 3 or 4 substituents Y, which can be the same or different, selected from the group consisting of methyl, ethyl, propyl, methoxy, ethoxy, hydroxy, hydroxymethyl, hydroxyethyl, chloro, iodo, bromo, fluoro, trifluoromethyl, trifluoromethoxy, methylsulfonyl, phenyl and cyano, or R represents naphtyl,
   or R represents a C₁₋₈branched or linear alkyl group, a C₃₋₇cycloalkyl group, C₃₋₇ cycloalkyl-C₁₋₂ alkyl group, a C₃₋₈ branche d or linear heteroalkyl group, a C₅₋₇ heterocycloalkyl group or a C₅₋₇ heterocycloalkyl -C₁₋₂ alkyl group which groups may be substituted with a fluoro atom or a CF₃ 3 or OH group,
- R₂ and R₃ are the same or different and represent H, a C₁₋₅ branched or linear alkyl group which alkyl group may be substituted with a hydroxy group, or 1-3 fluoro atoms or,
   R₂ represents a branched or linear C₁₋₃ alkoxy group, with the proviso that R₃ represents H or a methyl group
- R₂ and R₃ - together with the nitrogen atom to which they are bonded - form a saturated or unsaturated non-aromatic, monocyclic or bicyclic, heterocyclic group having 5 to 10 ring atoms which heterocyclic group contains one or two ring heteroatoms from the group (N, O, S), which heteroatoms can be the same or different, which heterocyclic group may be substituted with a C₁₋₃ alkyl group, a hydroxy group, or a fluoro atom,
- R₄ represents H or a methyl, ethyl, propyl, isopropyl or n-butyl group,
- X represents one of the subgroups (i), or (ii), 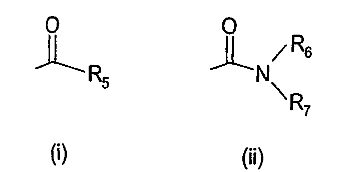 wherein:
- R₅ represents a hydrogen atom, or a C₁₋₈ branched or linear alkyl group, C₁₋₃-alkyl-SO₂-C₁₋₄- alkyl group, C₃₋₇ cycloalkyl group, C₃₋₇-cycloalkyl-C₁₋₂-alkyl group, C₅₋₇-heterocycloalkyl-C₁₋₂-alkyl group which groups may be substituted with a hydroxy, methyl or trifluoromethyl group or a fluoro atom and which C₅₋₇" heterocycloalkyl-C₁₋₂-alkyl group contains one or two heteroatoms from the group (O, N, S), or R₅ represents a phenyl, benzyl, phenethyl or phenylpropyl group which may be substituted on their phenyl ring with 1-3 substituents Y, wherein Y has the abovementioned meaning, or R₅ represents a pyridyl or thienyl group,
- R₆ represents a hydrogen atom or a branched or linear C₁₋₃ alkyl group,
- R₇ represents hydrogen, a branched or linear C ₁₋₈ alkyl or C₃₋₈-cycloalkyl-C₁₋₂-alkyl group, branched or linear C₁₋₈ alkoxy, C₃₋₈ cycloalkyl, C₅₋₁₀ bicycloalkyl, C₅₋₁₀ bicycloalkyl-C₁₋₂ alkyl, C ₆₋₁₀ tricycloalkyl, C ₆₋₁₀ tricycloalkylmethyl, which groups may contain one or more heteroatoms from the group (O, N, S) and which groups may be substituted with a hydroxy group, 1-3 methyl groups, an ethyl group or 1 - 3 fluoro atoms, or R₇ represents a phenyl, phenylamino, phenoxy, benzyl, phenethyl or phenylpropyl group, optionally substituted on their phenyl ring with 1-3 substituents Y, wherein Y has the abovementioned meaning, or R₇ represents a pyridyl or thienyl group, or R₇ represents a group NR₈R₉ wherein
   R₈ and R₉ - together with the nitrogen atom to which they are attached -form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, hydroxy or trifluoromethyl group or a fluoro atom, or
   R₆ and R₇ - together with the nitrogen atom to which they are attached - form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, amino, hydroxy or trifluoromethyl group or a fluoro atom, and pharmacologically acceptable salts thereof.

To the invention belong all compounds having formula (I), racemates, mixtures of diastereomers and the individual stereoisomers. Thus compounds in which the substituents on potentially asymmetrical carbon atoms are in either the R-configuration or the S-configuration belong to the invention.

The invention particularly relates to compounds having formula (I) wherein X represents the subgroup (ii), and all other symbols have the meanings as described above.

More particular the invention relates to compounds of formula (I) wherein X represents the subgroup (ii), and R represents phenyl, thienyl, pyridinyl, pyrimidinyl, pyrazinyl, pyridazinyl or triazinyl, which groups may be substituted with 1, 2, 3 or 4 substituents Y, which can be the same or different, from the group methyl, ethyl, propyl, methoxy, ethoxy, hydroxy, hydroxymethyl, hydroxyethyl, chloro, iodo, bromo, fluoro, trifluoromethyl, trifluoromethoxy, methylsulfonyl, phenyl or cyano, or R represents naphtyl, and all other symbols have the meanings as described above.

### GENERAL ASPECTS OF SYNTHESES

Compounds of formula (I) and the synthetic intermediates of formula (II) and (VI) respectively may be prepared by a variety of methodologies. The synthesis of structurally related imidazoles as cannabinoid receptor modulators has been described in WO 03/027076 and WO 03/063781. The synthesis of structurally related imidazoles as anti-obesity compounds has also been described in WO 03/040107. The selection of the particular method depends on factors such as the compatibility of functional groups with the reagents used, the possibility to use protecting groups, catalysts, activating and coupling reagents and the ultimate structural features present in the final compound being prepared. More information on activating and coupling methods of amines to carboxylic acids can be found in:
a) M. Bodanszky and A. Bodanszky: The Practice of Peptide Synthesis, Springer-Verlag, New York, 1994; ISBN: 0-387-57505-7;
b) K. Akaji et al., Tetrahedron Lett., 1994, 35, 3315-3318);
c) F. Albericio et al., Tetrahedron Lett.,1997, 38, 4853-4856).

More information on trimethylaluminum Al(CH₃)₃ promoted amidation reactions of esters can be found in: J. I. Levin, E. Turos, S. M. Weinreb, Synth Commun. 1982, 12, 989-993.). For more information on nucleophiles, electrophiles and the leaving group concept see: M. B. Smith and J. March: Advanced organic chemistry, p. 275, 5th ed., (2001) John Wiley & Sons, New York, ISBN: 0-471-58589-0). More information on addition and subsequent removal of protective groups in organic synthesis can be found in: T.W. Greene and P.G.M. Wuts, "Protective Groups in Organic Synthesis", third edition, John Wiley & Sons, Inc., New York, 1999.

A suitable synthesis for the compounds of the invention is the following:

### Synthesis route A

Reaction of a compound having formula (II) 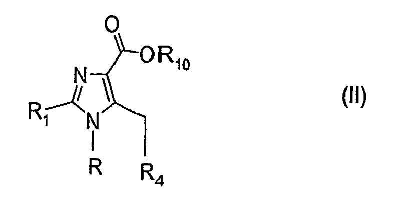 wherein R, R₁, R₄ have the meanings given above and R₁₀ represents a branched or linear alkyl group (C₁₋₄) or benzyl group with a regioselective brominating compound such as N-bromosuccinimide (NBS) in an organic solvent such as CCl₄ in the presence of a free-radical initiator such as dibenzoyl peroxide can give a compound of formula (III) 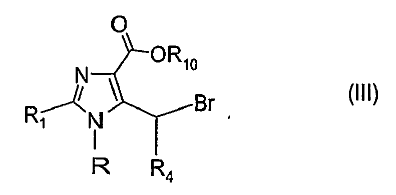 wherein R, R₁, R₄ and R₁₀ have the meanings given above. Reaction of a compound having formula (III) with an amine of general formula R₂R₃NH can give a compound of general formula (IV) 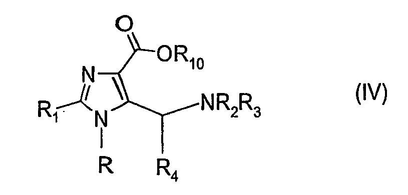 wherein R, R₁, R₂, R₃, R₄ and R₁₀ have the meanings given above.

Compounds of general formula (IV) which have been obtained according to synthesis route A can be converted to compounds of general formula (I) wherein X represents subgroup (ii), analogously to the procedures described in WO 03/027076.

### Synthesis route B

Reaction of a compound having formula (IV) wherein R, R₁, R₂, R₃ and R₄ have the meanings given above and R₁₀ represents a hydrogen atom 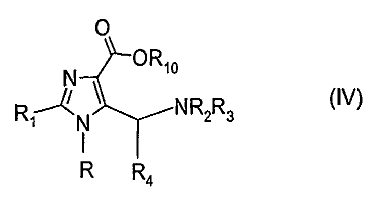 with N-methoxy-N-methylamine can give a compound of formula (V), 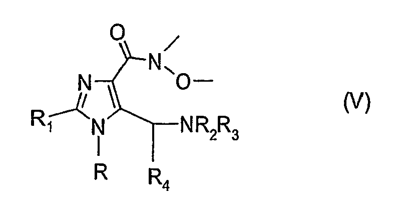 wherein R, R₁, R₂, R₃ and R₄ have the meanings given above. Reaction of a compound having formula (V) with an organometallic compound of general formula R₅-MgBr (a Grignard reagent) or R₅-Li (an organo -lithium reagent) can give a compound of general formula (I), wherein X represents subgroup (i). Such a reaction is preferably carried out under N₂ in an anhydrous inert organic solvent.

### Synthesis route C

2-Aryl-(1H)-imidazole-4-carboxylates can be obtained according to the procedure described in Tetrahedron Lett. (1971), 18, 1439-1440 (Heindel and Chun). Subsequent derivatisation (for example, an N-arylation or N-alkylation reaction) of the proper imidazole N-atom can produce compounds having formula (VI). Reaction of a compound having formula (VI) 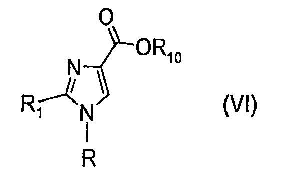 wherein R and R₁ have the meanings given above and R₁₀ represents a branched or linear alkyl group (C₁₋₄) or benzyl group, with a non-nucleophilic base such as lithium diisopropylamide (LDA), followed by an electrophile such as formic acid ethyl ester in an inert anhydrous organic solvent such as THF can give a compound of general formula (VII) 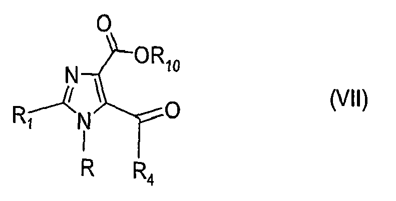 wherein R, R₁, R₄ and R₁₀ have the meanings given above in this synthetic route. Reductive amination of a compound having general formula (VII) with an amine of general formula R₂R₃NH in the presence of a reducing agent such as NaCNBH₃ can give a compound of general formula (IV) 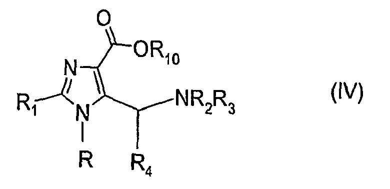 wherein R, R₁, R₂, R₃, R₄ and R₁₀ have the meanings given above in this synthetic route.

Compounds of general formula (IV) which have been obtained according to synthesis route C can be converted to compounds of general formula (I) wherein X represents subgroup (ii), analogously to the procedures described in WO 03/027076.

Pharmaceutically acceptable salts may be obtained using standard procedures well known in the art, for example by mixing a compound of the present invention with a suitable acid, for instance an inorganic acid such as hydrochloric acid, or with an organic acid.

Due to their cannabinoid receptor modulating activity the compounds according to the invention are suitable for use in the treatment of psychiatric disorders such as psychosis, anxiety, depression, attention deficits, memory disorders, cognitive disorders, appetite disorders, obesity, in particular juvenile obesity and drug induced obesity, addiction, appetence, drug dependence and neurological disorders such as neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, sexual disorders, liver cirrhosis, gastric ulcers, diarrhoea and cardiovascular disorders.

The cannabinoid receptor modulating activity of the compounds of the invention makes them particularly useful in the treatment of obesity, juvenile obesity and drug induced obesity, when used in combination with lipase inhibitors. Specific examples of compounds which can be used in such combination preparations are (but not restricted to) the synthetic lipase inhibitor orlistat, lipase inhibitors isolated from micro organisms such as lipstatin (from *Streptomyces toxytricini*), ebelactone B (from *Streptomyces aburaviensis*), synthetic derivatives of these compounds, as well as extracts of plants known to possess lipase inhibitory activity, for instance extracts of *Alpinia officinarum* or compounds isolated from such extracts like 3-methylethergalangin (from *A. officinarum*)*.*

The compounds of the invention can be brought into forms suitable for administration by means of usual processes using auxiliary substances and/or liquid or solid carrier materials.

Compounds of the invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge in the art. In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufact ure, use, or sale for human or veterinary administration.

### PHARMACOLOGICAL METHODS

### In vitro affinity for cannabinoid-CB₁ receptors

The affinity of the compounds of the invention for cannabinoid CB ₁ receptors can be determined using membrane preparations of Chinese hamster ovary (CHO) cells in which the human cannabinoid CB₁ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro affinity for cannabinoid-CB₂ receptors

The affinity of the compounds of the invention for cannabinoid CB₂ receptors can be determined using membrane preparations of Chinese hamster ovary (CHO) cells in which the human cannabinoid CB₂ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro cannabinoid-CB₁ receptor antagonism

In vitro CB₁ receptor antagonism can be assessed with the human CB₁ receptor cloned in Chinese hamster ovary (CHO) cells. CHO cells are grown in a Dulbecco's Modified Eagle's medium (DMEM) culture medium, supplemented with 10% heat-inactivated fetal calf serum. Medium is aspirated and replaced by DMEM, without fetal calf serum, but containing [³H]-arachidonic acid and incubated overnight in a cell culture stove (5% CO₂/95% air; 37 °C; water-saturated atmosphere). During this period [³H]-arachidonic acid is incorporated in membrane phospholipids. On the test day, medium is aspirated and cells are washed three times using 0.5 mL DMEM, containing 0.2% bovine serum albumin (BSA). Stimulation of the CB₁ receptor by WIN 55,212-2 leads to activation of PLA₂ followed by release of [³H]-arachidonic acid into the medium. This WIN 55,212-2-induced release is concentration-dependently antagonized by CB₁ receptor antagonists.

### In vivo cannabinoid-CB₁ receptor antagonism

In vivo CB₁ antagonism can be asse ssed with the CP-55,940-induced hypotension test in rat. Male normotensive rats (225-300 g; Harlan, Horst, The Netherlands) are anaesthetized with pentobarbital (80 mg/kg ip). Blood pressure is measured, via a cannula inserted into the left carotid artery, by means of a Spectramed DTX -plus pressure transducer (Spectramed B.V., Bilthoven, The Netherlands). After amplification by a Nihon Kohden Carrier Amplifier (Type AP-621G; Nihon Kohden B.V., Amsterdam, The Netherlands), the blood pressure signal is registered on a personal computer (Compaq Deskpro 386s), by means of a Po-Ne-Mah data-acquisition program (Po-Ne-Mah Inc., Storrs, USA). Heart rate is derived from the pulsatile pressure signal. All compounds are administered orally as a microsuspension in 1% methylcellulose 30 minutes before induction of the anesthesia which is 60 minutes prior to administration of the CB₁ receptor agonist CP-55,940. The injection volume is 10 ml/kg. After haemodynamic stabilization the CB₁ receptor agonist CP-55,940 (0.1 mg/kg *i.v*.) is administered and the hypotensive effect established. (Wagner, J. A.; Jarai, Z.; Batkai, S.; Kunos, G. Hemodynamic effects of cannabinoids: coronary and cerebral vasodilation mediated by cannabinoid CB1 receptors. Eur. J. Pharmacol. 2001, 423, 203-210); Lange, J. H. M. et al., J. Med. Chem. 2004,47,627-643.

### In vivo cannabinoid-CB₁ receptor (partial) agonistic activity

Cannabinoid receptor agonistic or partial agonistic activity of compounds of the invention can be determined according to published methods, such as assessment of in vivo cannabimimetic effects (Wiley, J. L. et al., J. Pharmacol. Exp. Ther. 2001, 296, 1013).

### DETERMINATION OF AQUEOUS SOLUBILITY

The compounds are dissolved in DMSO to give 10 mg/ml stock solutions. These stock solutions are divided over several plates. Several diluted concentrations are made with DMSO to 5, 2.5, 1.25, 0.625 and 0.3125 mg/ml, respectively. The stock solution and each derived concentration from this stock solution is taken and diluted 100 fold with water or with water containing a buffer. In every case this gives an aqueous solution which contains 1 % DMSO (v/v) and 100, 50, 25,12.5 and 6.25 and 3.125 µg compound/ml, respectively. The formation of a precipitate is determined in each plate. The measurements were performed at pH= 7, applying a HEPES buffer and at pH= 2, applying hydrochloric acid. Aqueous solubility is expressed in µg/ml in the table below.

The specific compounds of which the synthesis is described below are intended to further illustrate the invention in more detail, and therefore are not deemed to restrict the scope of the invention in any way.

### EXAMPLE 1: SYNTHESES OF SPECIFIC COMPOUNDS

### Synthesis of compound 1

**Part A: Ethyl 5-bromomethyl-1-(4-chlorophenyl)-2-(2,4-dichlorophenyl)-1H-imidazole-4-carboxylate.** To a magnetically stirred mixture of ethyl 1-(4-chlorophenyl)-2-(2,4-dichlorophenyl)-5-methyl-1H-imidazole-4-carboxylate (2.05 g, 5.00 mmol) in CCl₄ (25 mL) was added N-bromosuccinimide (NBS) (1.34 g, 7.53 mmol) and dibenzoyl peroxide (10.0 mg, assay 75%, 0.0310 mmol) and the resulting mixture was refluxed for 38 h. The formed precipitate was removed by filtration. The filtrate was successively washed with brine and water, dried over MgSO₄, filtered and concentrated in vacuo. The residue was purified by flash chromatography (CH₂Cl₂/acetone = 98/2 (v/v)) to give ethyl 5-bromomethyl-1-(4-chlorophenyl)-2-(2,4-dichlorophenyl)-1H-imidazole-4-carboxylate (1.29 g, 53 % yield) as an amorphous solid, ¹H-NMR (200 MHz, CDCl₃): δ 1.45 (J = 7 Hz, 3H), 4.48 (q, J = 7 Hz, 2H), 4.72 (s, 2H), 7.18-7.43 (m, 7H).

**Part B:** To a magnetically stirred solution of ethyl 5-bromomethyl-1-(4-chlorophenyl)-2-(2,4-dichlorophenyl)-1H-imidazole-4-carboxylate (11.69 g, 23.9 mmol) in acetonitrile (40 ml) is added diisopropylethylamine (DIPEA) (5.2 ml, 30 mmol) and pyrrolidine (1.7 g, 23.9 mmol) and the resulting solution is reacted at room temperature for 2 hours. Water (250 ml) is added and the resulting mixture is extracted three times with dichloromethane. The collected dichloromethane layers are dried over Na ₂SO₄, filtered and concentrated in vacuo. Subsequent purification with flash chromatography (petroleu m ether/EtOAc = 20/80 (v/v)) gives ethyl 1-(4-chlorophenyl)-2-(2,4-dichloro-phenyl)-5-(pyrrolidin-1-ylmethyl)-1H-imidazole-4-carboxylate (3.7 gram, 32 % yield). ¹H-NMR (200 MHz, CDCl₃): δ1.42 (t, J = 7 Hz, 3H), 1.62-1.72 (m, 4H), 2.42-2.50 (m, 4H), 3.83 (s, 2H), 4.43 (q, J = 7 Hz, 2H), 7.17-7.40 (m, 7H).

**Part C**: Cyclohexylamine (0.91 ml, 8 mmol) is dissolved in dichloromethane (20 ml) and (CH₃)₃Al (4 ml of a 2 M solution in heptane, 8 mmol) is added. The resulting mixture is stirred for 10 minutes at room temperature and ethyl 1-(4-chlorophenyl)-2-(2,4-dichlorophenyl)-5-(pyrrolidin-1-ylmethyl)-1H-imidazole-4-carboxylate (1.8 gram , 3.8 mmol) is added. The resulting mixture is stirred at room temperature for 16 hours, poured into an aqueous NaHCO₃ solution , stirred for 30 minutes and filtered over hyflo. The filtrated is twice extracted with dichloromethane. The organic layers are dried over Na₂SO₄, filtered and concentrated in vacuo. Subsequent purification with flash chromatography (dichloromethane/methanol = 98/2 (v/v)) gives N-cyclohexyl-1-(4-chlorophenyl)-2-(2,4-dichlorophenyl)-5-(pyrrolidin-1-ylmethyl)-1H-imidazole-4-carboxamide, compound 1 (1.06 gram, 53 % yield). Melting point: 155-157 °C.

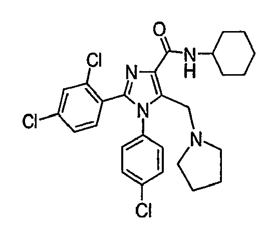

Analogously, the following **compounds 2 - 6** were prepared :
2. N-cyclohexyl-1-(4-chlorophenyl)-2-(2,4-dichlorophenyl)-5-(N-ethyl,N-methylaminomethyl)-1H-imidazole-4-carboxamide. Melting point: 135-136 °C. 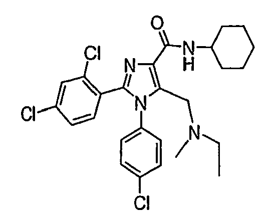
3. N-(piperidin-1-yl)-1-(4-chlorophenyl)-2-(2,4-dichlorophenyl)-5-(N,N-dimethylaminomethyl)-1H-imidazole-4-carboxamide. Melting point: 163-166 °C. 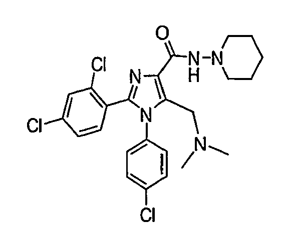
4. N-cyclohexyl-1-(4-chlorophenyl)-2-(2,4-dichlorophenyl)-5-(N,N-dimethylaminomethyl)-1H-imidazole-4-carboxamide. ¹H-NMR (400 MHz, CDCl₃): δ1.12-1.68 (m, 6H), 1.72-1.80 (m, 2H), 1.99-2.06 (m, 2H), 2.18 (s, 6H), 3.65 (s, 2H), 3.88-4.00 (m, 1H), 7.22-7.37 (m, 8H). 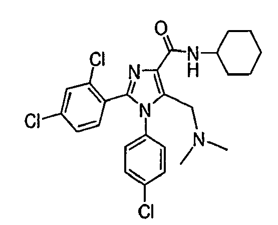
5. N-(piperidin-1-yl)-1-(4-chlorophenyl)-2-(2,4-dichlorophenyl)-5-(pyrrolidin-1-ylmethyl)-1H-imidazole-4-carboxamide. 2 HCl. ¹H-NMR (400 MHz, DMSO-d₆): δ 1.46-1.56 (m, 2H), 1.79-1.96 (m, 8H), 2.90-3.00 (m, 2H), 3.26-3.50 (m, 6H), 4.57 (br s, 2H), 7.45-7.60 (m, 6H), 7.70 (d, J = 8 Hz, 1H), 10.95 (br s, 2H). 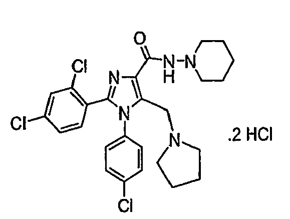
6. N-(piperidin-1-yl)-1-(4-chlorophenyl)-2-(2,4-dichlorophenyl)-5-(N-ethyl,N-methylaminomethyl)-1H-imidazole-4-carboxamide. ¹H-NMR (400 MHz, CDCl₃): δ 0.86 (t, J = 7 Hz, 3H), 1.38-1.46 (m, 2H), 1.72-1.78 (m, 4H), 2.14 (s, 3H), 2.36 (q, J = 7 Hz, 2H), 2.82-2.86 (m, 4H), 3.72 (s, 2H), 7.21-7.31 (m, 6H), 7.34 (d, J = 2 Hz, 1H), 8.16 (br s, 1H).

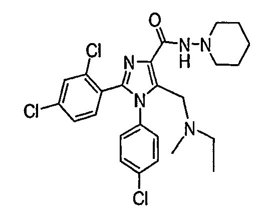

### EXAMPLE 2: DETERMINATION OF AQUEOUS SOLUBILITIES

Aqueous solubilities of two of the compounds of general formula (I), the compounds 1 and 4, and the compound 1-(4-chlorophenyl)-2-(2,4-dichloro-phenyl)-N-cyclohexyl-1H-imidazole-4-carboxamide (compound 3 from WO 03/063781) were determined according to the procedure described above. The results are shown in Table 1.

**Table 1: aqueous solubility (expressed in µg/ml)**

| **Compound name** | **Aqueous solubility in µg/ml at:** | |
|---|---|---|
| | **pH=2** | **pH=7** |
| | | |
| N-cyclohexyl-1-(4-chlorophenyl)-2-(2,4-dichlorophenyl)-5-(pyrrolidin-1-ylmethyl)-1H-imidazole-4-carboxamide *(compound 1)* | **> 100** | **50** |
| N-cyclohexyl-1-(4-chlorophenyl)-2-(2,4-dichlorophenyl)-5-(N,N-dimethylaminomethyl)-1H-imidazole-4-carboxamide (*compound 4*) | **> 100** | **50** |
| N-cyclohexyl-1-(4-chlorophenyl)-2-(2,4-dichlorophenyl)-1H-imidazole-4-carboxamide *(*WO 03/063781*)* | **25** | **25** |

### EXAMPLE 3: PHARMACOLOGICAL DATA

Pharmacological test results of a subset of the compounds of the invention, obtained with the assay described above, are given in table 2 below:

**Table 2: pharmacological data**

| | Human cannabinoid-CB₁ receptor |
|---|---|
| | *In vitro* affinity - pKᵢ value |
| Compound | |
| | |
| Compound 1 | **8.1** |
| Compound 2 | **8.2** |
| Compound 3 | **7.0** |
| Compound 4 | **7.5** |
| Compound 5 | **6.3** |
| Compound 6 | **6.9** |

## Claims

1. Compounds of the general formula (I) 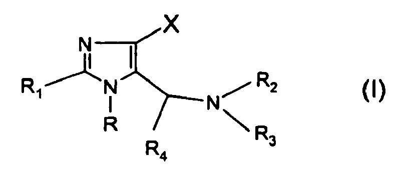 wherein:
- R and R₁ are the same or different and represent phenyl, thienyl, pyridinyl, pyrimidinyl, pyrazinyl, pyridazinyl or triazinyl, which groups may be substituted with 1, 2, 3 or 4 substituents Y, which can be the same or different, selected from the group consisting of methyl, ethyl, propyl, methoxy, ethoxy, hydroxy, hydroxymethyl, hydroxyethyl, chloro, iodo, bromo, fluoro, trifluoromethyl, trifluoromethoxy, methylsulfonyl, phenyl and cyano, or R represents naphtyl, or R represents a C₁₋₈ branched or linear alkyl group, a C₃₋₇ cycloalkyl group, C₃₋₇ cycloalkyl-C₁₋₂ alkyl group, a C₃₋₈ branched or linear heteroalkyl group, a C₅₋₇ heterocycloalkyl group or a C₅₋₇ heterocycloalkyl-C₁₋₂ alkyl group which groups may be substituted with a fluoro atom or a CF₃ or OH group,
- R₂ and R₃ are the same or different and represent H, a C₁₋₅ branched or linear alkyl group which alkyl group may be substituted with a hydroxy group, or 1-3 fluoro atoms or,
R₂ represents a branched or linear C₁₋₃ alkoxy group, with the proviso that R₃ represents H or a methyl group, or
R₂ and R₃ - together with the nitrogen atom to which they are bonded - form a saturated or unsaturated non-aromatic, monocyclic or bicyclic, heterocyclic group having 5 to 10 ring atoms which heterocyclic group contains one or two ring heteroatoms from the group (N, O, S), which heteroatoms can be the same or different, which heterocyclic group may be substituted with a C₁₋₃ alkyl group, a hydroxy group, or a fluoro atom,
- R₄ represents H or a methyl, ethyl, propyl, isopropyl or n-butyl group,
- X represents one of the subgroups (i), or (ii), 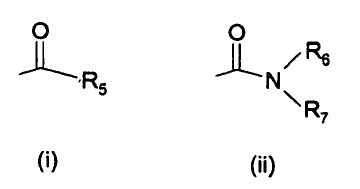
wherein:
- R₅ represents a hydrogen atom, or a C₁₋₈ branched or linear alkyl group, C₁₋₃-alkyl-SO₂-C₁₋₄- alkyl group, C₃₋₇ cycloalkyl group, C₃₋₇-cycloalkyl-C₁₋₂-alkyl group, C₅₋₇-heterocycloalkyl-C₁₋₂-alkyl group which groups may be substituted with a hydroxy, methyl or trifluoromethyl group or a fluoro atom and which C₅₋₇-heterocycloalkyl-C₁₋₂-alkyl group contains one or two heteroatoms from the group (O, N, S), or R₅ represents a phenyl, benzyl, phenethyl or phenylpropyl group which may be substituted on their phenyl ring with 1-3 substituents Y, wherein Y has the abovementioned meaning, or R₅ represents a pyridyl or thienyl group,
- R₆ represents a hydrogen atom or a branched or linear C₁₋₃ alkyl group,
- R₇ represents hydrogen, a branched or linear C₁₋₈ alkyl or C₃₋₈-cycloalkyl-C₁₋₂-alkyl group, branched or linear C₁₋₈ alkoxy, C₃₋₈ cycloalkyl, C₅₋₁₀ bicycloalkyl, C₅₋₁₀ bicycloalkyl-C₁₋₂ alkyl, C₆₋₁₀ tricycloalkyl, C₆₋₁₀ tricycloalkylmethyl, which groups may contain one or more heteroatoms from the group (O, N, S) and which groups may be substituted with a hydroxy group, 1-3 methyl groups, an ethyl group or 1-3 fluoro atoms, or R₇ represents a phenyl, phenylamino, phenoxy, benzyl, phenethyl or phenylpropyl group, optionally substituted on their phenyl ring with 1-3 substituents Y, wherein Y has the abovementioned meaning, or R₇ represents a pyridyl or thienyl group, or R₇ represents a group NR₈R₉ wherein
- R₈ and R₉ - together with the nitrogen atom to which they are attached -form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, hydroxy or trifluoromethyl group or a fluoro atom, or
- R₆ and R₇ - together with the nitrogen atom to which they are attached - form a saturated or unsaturated, monocyclic or bicyclic, heterocyclic group having 4 to 10 ring atoms, which heterocyclic group contains one or more heteroatoms from the group (O, N, S) and which heterocyclic group may be substituted with a branched or linear C₁₋₃ alkyl, phenyl, amino, hydroxy or trifluoromethyl group or a fluoro atom,
and all stereoisomers, and pharmacologically acceptable salts thereof.

2. Compounds as claimed in claim 1 having general formula (I) wherein X represents the subgroup (ii), and all other symbols have the meanings as given in claim 1.

3. Compounds as claimed in claim 1 having formula (I) wherein X represents the subgroup (ii), R represents phenyl, thienyl, pyridinyl, pyrimidinyl, pyrazinyl, pyridazinyl or triazinyl, which groups may be substituted with 1, 2, 3 or 4 substituents Y, which can be the same or different, from the group methyl, ethyl, propyl, methoxy, ethoxy, hydroxy, hydroxymethyl, hydroxyethyl, chloro, iodo, bromo, fluoro, trifluoromethyl, trifluoromethoxy, methylsulfonyl, phenyl or cyano, or R represents naphtyl, and all other symbols have the meanings as given in claim 1.

4. Compounds of the general formula (IV) 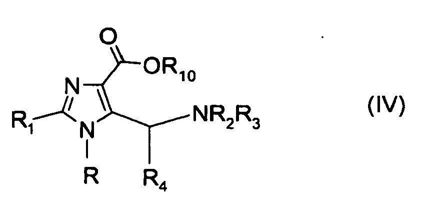 wherein R, R₁, R₂, R₃ and R₄ have the meanings given in claim 1 and R₁₀ represents a hydrogen atom, a linear or branched C₁₋₄ alkyl group or a benzyl group, such compounds being useful in the synthesis of compounds of the general formula (I).

5. Compounds of the general formula (VII) 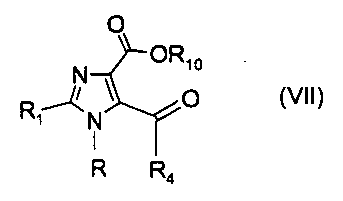 wherein R₁ has the meanings given in claim 1, R₄ represents a methyl, ethyl, propyl, isopropyl or n-butyl group, and R represents a C₁₋₈ branched or linear alkyl group, a C₃₋₇ cycloalkyl group, C₃₋₇ cycloalkyl-C₁₋₂ alkyl group, a C₃₋₈ branched or linear heteroalkyl group, a C₅₋₇ heterocycloalkyl group or a C₅₋₇ heterocycloalkyl-C₁₋₂ alkyl group which groups may be substituted with a fluoro atom or a CF₃ or OH group, and R₁₀ represents a methyl, n-propyl, isopropyl, n-butyl, 2-butyl, isobutyl or 2-methyl-n-propyl or a benzyl group.

6. Pharmaceutical compositions containing at least one compound as claimed in any one of the claims 1-3 as an active component.

7. A compound as claimed in any of the claims 1-3, or a salt thereof, for use as a medicament

8. Use of a compound as claimed in any one of the claims 1-3 for the preparation of a pharmaceutical composition for the treatment of disorders involving cannabinoid neurotransmission.

9. Use as claimed in claim 8, **characterized in that** said disorders are psychosis, anxiety, depression, attention deficits, memory disorders, cognitive disorders, appetite disorders, obesity, in particular juvenile obesity and drug induced obesity, addiction, appetence, drug dependence and neurological disorders such as neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, sexual disorders, liver cirrhosis, gastric ulcers, diarrhoea and cardiovascular disorders.

10. Use as claimed in claim 8, **characterized in that** said disorders are eating disorders, in particular obesity, juvenile obesity and drug induced obesity.

11. Use of a compound as claimed in claim 1 for the preparation of a pharmaceutical composition for the treatment of eating disorders, in particular obesity, juvenile obesity and drug induced obesity, **characterized in that** said pharmaceutical composition also contains at least one lipase inhibitor.

12. Use as claimed in claim 11, **characterized in that** said lipase inhibitor is orlistat or lipstatin.

## Patentansprüche

1. Verbindungen der allgemeinen Formel (I) 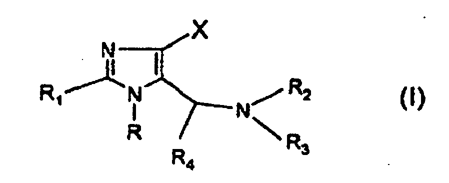
- worin R und R₁ gleich oder verschieden sind und Phenyl, Thienyl, Pyridinyl, Pyrimidinyl, Pyrazinyl, Pyridazinyl oder Triazinyl darstellen, welche Gruppen mit 1, 2, 3 oder 4 Substituenten Y substituiert sein können, die gleich oder verschieden sein können, ausgewählt aus der Gruppe bestehend aus Methyl, Ethyl, Propyl, Methoxy, Ethoxy, Hydroxy, Hydroxymethyl, Hydroxyethyl, Chlor, Iod, Brom, Fluor, Trifluormethyl, Trifluormethoxy, Methylsulfonyl, Phenyl und Cyano, oder R Naphtyl darstellt, oder R eine C₁₋₈ verzweigte oder lineare Alkylgruppe, eine C₃₋₇-Cycloalkylgruppe, C₃₋₇-Cycloalkyl-C₁₋₂-alkylgruppe, eine verzweigte oder lineare C₃₋₈-Heteroalkylgruppe, eine C₅₋₇-Heterocycloalkylgruppe oder eine C₅₋₇-Heterocycloalkyl-C₁₋₂-alkylgruppe darstellt, welche Gruppen mit einem Fluoratom oder einer CF₃- oder OH-Gruppe substituiert sein können,
- R₂ und R₃ gleich oder verschieden sind und H, eine C₁₋₅ verzweigte oder lineare Alkylgruppe darstellen, welche Alkylgruppe mit einer Hydroxygrupe oder 1-3 Fluoratomen subsituiert sein kann, oder
R₂ eine verzweigte oder lineare C₁₋₃-Alkoxygruppe darstellt, unter der Voraussetzung, dass R₃ H oder eine Methylgruppe darstellt, oder
R₂ und R₃ - zusammen mit dem Stickstoffatom, an das sie gebunden sind - eine gesättigte oder ungesättigte, nichtaromatische, monocyclische oder bicyclische, heterocyclische Gruppe mit 5 bis 10 Ringatomen bilden, wobei die heterocyclische Gruppe ein oder zwei Ring-Heteroatome aus der Gruppe (N, O, S) enthält, welche Heteroatome gleich oder verschieden sein können, welche heterocyclische Gruppe mit einer C₁₋₃-Alkylgruppe, einer Hydroxygruppe oder einem Fluoratom substituiert sein kann,
- R₄ H oder eine Methyl-, Ethyl-, Propyl-, Isopropyl- oder n-Butylgruppe darstellt,
- X eine der Untergruppen (i) oder (ii) darstellt, 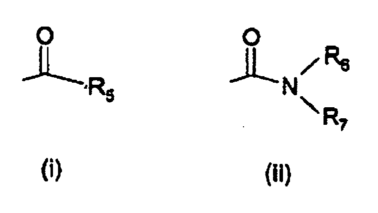 worin
- R₅ ein Wasserstoffatom oder eine C₁₋₈ verzweigte oder lineare Alkylgruppe, C₁₋₃-Alkyl-SO₂-C₁₋₄-alkylgruppe, C₃₋₇-Cycloalkylgruppe, C₃₋₇-Cycloalkyl-C₁₋₂-alkylgruppe, C₅₋₇-Heterocycloalkyl-C₁₋₂-alkylgruppe darstellt, welche Gruppen mit einer Hydroxy-, Methyl- oder Trifluormethylgruppe oder einem Fluoratom substituiert sein können, und welche C₅₋₇-Heterocycloalkyl-C₁₋₂-alkylgruppe ein oder zwei Heteroatome aus der Gruppe (O, N, S)enthält, oder R₅ eine Phenyl-, Benzyl-, Phenethyl- oder Phenylpropylgruppe darstellt, die an ihrem Phenylring mit 1-3 Substituenten Y substituiert sein kann, wobei Y die oben genannte Bedeutung hat, oder R₅ eine Pyridyl- oder Thienylgruppe darstellt,
- R₆ ein Wasserstoffatom oder eine verzweigte oder lineare C₁₋₃-Alkylgruppe darstellt,
- R₇ Wasserstoff, eine verzweigte oder lineare C₁₋₈-Alkyl- oder C₃₋₈-Cycloalkyl-C₁₋₂-alkylgruppe, verzweigtes oder lineares C₁₋₈-Alkoxy, C₃₋₈-Cycloalkyl, C₅₋₁₀-Bicycloalkyl, C₅₋₁₀-Bicycloalkyl-C₁₋₂-alkyl, C₆₋₁₀-Tricycloalkyl, C₆₋₁₀-Tricycloalkylmethyl darstellt, welche Gruppen ein oder mehrere Heteroatome aus der Gruppe (O, N, S) enthalten können, und welche Gruppen mit einer Hydroxygruppe, 1-3 Methylgruppen, einer Ethylgruppe oder 1-3 Fluoratomen substituiert sein können, oder R₇ eine Phenyl-, Phenylamino- , Phenoxy-, Benzyl-, Phenethyl- oder Phenylpropylgruppe darstellt, gegebenenfalls an ihrem Phenylring substituiert mit 1-3 Substituenten Y, wobei Y die oben genannte Bedeutung hat, oder R₇ eine Pyridyl- oder Thienylgruppe darstellt, oder R₇ eine Gruppe NR₈R₉ darstellt, worin
- R₈ and R₉ - zusammen mit dem Stickstoffatom, an das sie gebunden sind - eine gesättigte oder ungesättigte, monocyclische oder bicyclische, heterocyclische Gruppe mit 4 bis 10 Ringatomen bilden, wobei die heterocyclische Gruppe ein oder mehrere Heteroatome aus der Gruppe (O, N, S) enthält, und welche heterocyclische Gruppe mit einer verzweigten oder linearen C₁₋₃-Alkyl-, Phenyl-, Hydroxy- oder Trifluormethylgruppe oder einem Fluoratom substituiert sein kann, oder
- R₆ und R₇ - zusammen mit dem Stickstoffatom, an das sie gebunden sind - eine gesättigte oder ungesättigte, monocyclische oder bicyclische, heterocyclische Gruppe mit 4 bis 10 Ringatomen bilden, wobei die heterocyclische Gruppe ein oder mehrere Heteroatome aus der Gruppe (O, N, S) enthält, und welche heterocyclische Gruppe mit einer verzweigten oder linearen C₁₋₃-Alkyl-, Phenyl-, Amino-, Hydroxy- oder Trifluormethylgruppe oder einem Fluoratom substituiert sein kann,
und alle Stereoisomere und pharmakologisch annehmbaren Salze davon.

2. Verbindungen gemäß Anspruch 1 mit der allgemeinen Formel (I), worin X die Untergruppe (ii) darstellt und alle anderen Symbole die gleichen Bedeutungen wie in Anspruch 1 haben.

3. Verbindungen gemäß Anspruch 1 mit der Formel (I), worin X die Untergruppe (ii) darstellt, R Phenyl, Thienyl, Pyridinyl, Pyrimidinyl, Pyrazinyl, Pyridazinyl oder Triazinyl darstellt, welche Gruppen mit 1, 2, 3 oder 4 Substituenten Y substituiert sein können, die gleich oder verschieden sein können, aus der Gruppe Methyl, Ethyl, Propyl, Methoxy, Ethoxy, Hydroxy, Hydroxymethyl, Hydroxyethyl, Chlor, Iod, Brom, Fluor, Trifluormethyl, Trifluormethoxy, Methylsulfonyl, Phenyl oder Cyano, oder R Naphtyl darstellt und alle anderen Symbole die gleichen Bedeutungen haben wie in Anspruch 1.

4. Verbindungen der allgemeinen Formel (IV) 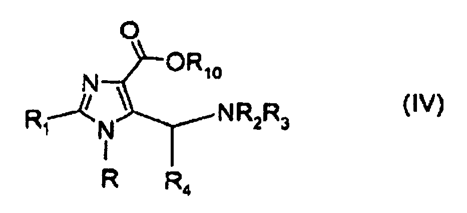 worin R, R₁, R₂, R₃ und R₄ die in Anspruch 1 genannten Bedeutungen haben und R₁₀ ein Wasserstoffatom, eine lineare oder verzweigte C₁₋₄-Alkylgruppe oder eine Benzylgruppe darstellt, wobei derartige Verbindungen in der Synthese von Verbindungen der allgemeinen Formel (I) nützlich sind.

5. Verbindungen der allgemeinen Formel (VII) 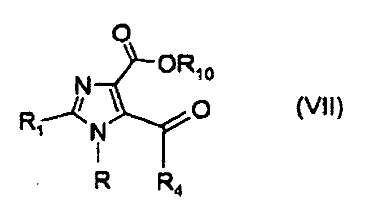 worin R₁ die in Anspruch 1 genannte Bedeutung hat, R₄ eine Methyl- , Ethyl-, Propyl-, Isopropyl oder n-Butylgruppe darstellt und R eine verzweigte oder lineare C₁₋₈-Alkylgruppe, eine C₃₋₇-Cycloalkylgruppe, C₃₋₇-Cycloalkyl-C₁₋₂-alkylgruppe, eine verzweigte oder lineare C₃₋₈-Heteroalkylgruppe, eine C₅₋₇-Heterocycloalkylgruppe oder eine C₅₋₇-Heterocycloalkyl-C₁₋₂-alkylgruppe darstellt, welche Gruppen mit einem Fluoratom oder einer CF₃- oder OH-Gruppe substituiert sein können, und R₁₀ eine Methyl-, n-Propyl-, Isopropyl-, n-Butyl-, 2-Butyl-, Isobutyl- oder 2-Methyl-n-propyl- oder eine Benzylgruppe darstellt.

6. Pharmazeutische Zusammensetzungen, die mindestens eine Verbindung gemäß einem der Ansprüche 1-3 als aktive Komponente enthalten.

7. Verbindung gemäß einem der Ansprüche 1-3 oder ein Salz davon zur Verwendung als Medikament.

8. Verwendung einer Verbindung gemäß einem der Ansprüche 1-3 zur Herstellung einer pharmazeutischen Zusammensetzung zur Behandlung von Störungen, die mit der Cannabinoid-Neurotransmission zusammenhängen.

9. Verwendung gemäß Anspruch 8, **dadurch gekennzeichnet, dass** die Störungen Psychose, Angstzustände, Depression, Aufmerksamkeitsstörungen, Gedächtnisstörungen, kognitive Störungen, Appetitstörungen, Fettleibigkeit, insbesondere juvenile Fettleibigkeit und von Drogen bzw. Medikamenten induzierte Fettleibigkeit, Sucht, Appetenz, Drogenabhängigkeit und neurologische Störungen, wie neurodegenerative Störungen, Demenz, Dystonie, Muskelspasmen, Tremor, Epilepsie, multiple Sklerose, traumatische Gehirnverletzungen, Schlaganfall, Morbus Parkinson, Morbus Alzheimer, Epilepsie, Huntington-Krankheit, Tourette-Syndrom, zerebrale Ischämie, Gehirnschlag, craniocerebrales Trauma, Schlaganfall, Wirbelsäulenverletzung, Nervenentzündungs-Störungen, Plaquesklerose, virale Enzephalitis, Störungen im Zusammenhang mit der Demyelinisation sind, sowie zur Behandlung von Schmerzstörungen einschließlich neuropathischen Schmerzstörungen und anderen Erkrankungen im Zusammenhang mit der Cannabinoid-Neurotransmission einschließlich Behandlung von septischem Schock, Glaukom, Krebs, Diabetes, Erbrechen, Übelkeit, Asthma, Atemwegserkrankungen, gastrointestinalen Störungen, sexuellen Störungen, Leberzirrhose, Magengeschwüren, Diarrhö und kardiovaskulären Störungen.

10. Verwendung gemäß Anspruch 8, **dadurch gekennzeichnet, dass** die Störungen Essstörungen sind, insbesondere Fettleibigkeit, juvenile Fettleibigkeit und von Drogen bzw. Medikamenten induzierte Fettleibigkeit.

11. Verwendung einer Verbindung gemäß Anspruch 1 zur Herstellung einer pharmazeutischen Zusammensetzung zur Behandlung von Essstörungen, insbesondere Fettleibigkeit, juveniler Fettleibigkeit und von Drogen bzw. Medikamenten induzierter Fettleibigkeit, **dadurch gekennzeichnet, dass** die pharmazeutische Zusammensetzung auch mindestens einen Lipaseinhibitor enthält.

12. Verwendung gemäß Anspruch 11, **dadurch gekennzeichnet, dass** der Lipaseinhibitor Orlistat oder Lipstatin ist.

## Revendications

1. Composés de formule générale (I) 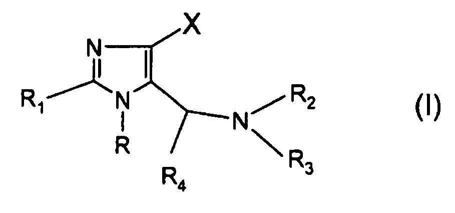 où :
- R et R₁ sont identiques ou différents et représentent phényle, thiényle, pyridinyle, pyrimidinyle, pyrazinyle, pyridazinyle ou triazinyle, lesquels groupes peuvent être substitués avec 1, 2, 3 ou 4 substituants Y, qui peuvent être identiques ou différents, choisis dans le groupe consistant en méthyle, éthyle, propyle, méthoxy, éthoxy, hydroxy, hydroxyméthyle, hydroxyéthyle, chloro, iodo, bromo, fluoro, trifluorométhyle, trifluorométhoxy, méthylsulfonyle, phényle et cyano, ou bien R représente naphtyle, ou bien R représente un groupe C₁₋₈ alkyle ramifié ou linéaire, un groupe C₃₋₇ cycloalkyle, un groupe C₃₋₇ cycloalkyl-C₁₋₂alkyle, un groupe C₃₋₈ hétéroalkyle ramifié ou linéaire, un groupe C₅₋₇ hétérocycloalkyle ou un groupe C₅₋₇ hétérocycloalkyl-C₁₋₂ alkyle, lesquels groupes peuvent être substitués avec un atome de fluor ou un groupe CF₃ ou OH,
- R₂ et R₃ sont identiques ou différents et représentent H, un groupe C₁₋₅ alkyle ramifié ou linéaire, lequel groupe alkyle peut être substitué avec un groupe hydroxy, ou 1-3 atomes de fluor ou
R₂ représente un groupe C₁₋₃ alcoxy ramifié ou linéaire, à condition que R₃ représente H ou un groupe méthyle, ou
R₂ et R₃, avec l'atome d'azote auquel ils sont liés, forment un groupe hétérocyclique, monocyclique ou bicyclique, non aromatique, saturé ou insaturé ayant 5 à 10 atomes cycliques, lequel groupe hétérocyclique contient un ou deux hétéroatomes cycliques du groupe (N, O, S), lesquels hétéroatomes peuvent être identiques ou différents, lequel groupe hétérocyclique peut être substitué avec un groupe C₁₋₃ alkyle, un groupe hydroxy ou un atome de fluor,
- R₄ représente H ou un groupe méthyle, éthyle, propyle, isopropyle ou n-butyle,
- X représente l'un des sous-groupes (i) ou (ii), 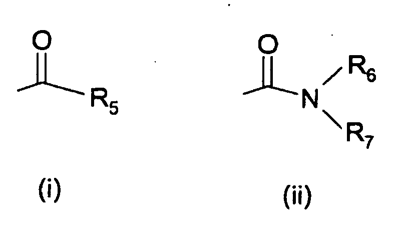 où :
- R₅ représente un atome d'hydrogène ou un groupe C₁₋₈ alkyle ramifié ou linéaire, un groupe C₁₋₃-alkyle-SO₂-C₁₋₄-alkyle, un groupe C₃₋₇ cycloalkyle, un groupe C₃₋₇-cycloalkyl-C₁₋₂-alkyle, un groupe C₅₋₇-hétérocycloalkyl-C₁₋₂-alkyle, lesquels groupes peuvent être substitués avec un groupe hydroxy, méthyle ou trifluorométhyle ou un atome de fluor et lequel groupe C₅₋₇-hétérocycloalkyl-C₁₋₂-alkyle contient un ou deux hétéroatomes du groupe (O, N, S), ou bien R₅ représente un groupe phényle, benzyle, phénéthyle ou phénylpropyle qui peuvent être substitués sur leur cycle phényle avec 1-3 substituants Y, où Y a la signification mentionnée ci-dessus, ou bien R₅ représente un groupe pyridyle ou thiényle,
- R₆ représente un atome d'hydrogène ou un groupe C₁₋₃ alkyle ramifié ou linéaire,
- R₇ représente l'hydrogène, un groupe C₁₋₈ alkyle ramifié ou linéaire ou C₃₋₈-cycloalkyle-C₁₋₂-alkyle, un groupe C₁₋₈ alcoxy ramifié ou linéaire, C₃₋₈-cycloalkyle, C₅₋₁₀ bicycloalkyle, C₅₋₁₀ bicycloalkyl-C₁₋₂-alkyle, C₆₋₁₀-tricycloalkyle, C₆₋₁₀-tricycloalkylméthyle, lesquels groupes peuvent contenir un ou plusieurs hétéroatomes du groupe (O, N, S) et lesquels groupes peuvent être substitués avec un groupe hydroxy, 1-3 groupes méthyle, un groupe éthyle ou 1-3 atomes de fluor, ou bien R₇ représente un groupe phényle, phénylamino, phénoxy, benzyle, phénéthyle ou phénylpropyle, éventuellement substitués sur leur cycle phényle avec 1-3 substituants Y, où Y a la signification mentionnée ci-dessus, ou bien R₇ représente un groupe pyridyle ou thiényle, ou bien R₇ représente un groupe NR₈R₉ où
- R₈ et R₉, avec l'atome d'azote auquel ils sont liés, forment un groupe hétérocyclique, monocyclique ou bicyclique, saturé ou insaturé ayant 4 à 10 atomes cycliques, lequel groupe hétérocyclique contient un ou plusieurs hétéroatomes du groupe (O, N, S) et lequel groupe hétérocyclique peut être substitué avec un groupe C₁₋₃ alkyle ramifié ou linéaire, phényle, hydroxy ou trifluorométhyle ou un atome de fluor, ou
- R₆ et R₇, avec l'atome d'azote auquel ils sont liés, forment un groupe hétérocyclique, monocyclique ou bicyclique, saturé ou insaturé, ayant 4 à 10 atomes cycliques, lequel groupe hétérocyclique contient un ou plusieurs hétéroatomes du groupe (O, N, S) et lequel groupe hétérocyclique peut être substitué avec un groupe C₁₋₃ alkyle ramifié ou linéaire, phényle, amino, hydroxy ou trifluorométhyle ou un atome de fluor, et tous leurs stéréoisomères, et leurs sels pharmacologiquement acceptables.

2. Composés selon la revendication 1 ayant la formule générale (I) où X représente le sous-groupe (ii), et tous les autres symboles ont les significations données dans la revendication 1.

3. Composés selon la revendication 1 ayant la formule (I) où X représente le sous-groupe (ii), R représente phényle, thiényle, pyridinyle, pyrimidinyle, pyrazinyle, pyridazinyle ou triazinyle, lesquels groupes peuvent être substitués avec 1, 2, 3 ou 4 substituants Y, qui peuvent être identiques ou différents, du groupe méthyle, éthyle, propyle, méthoxy, éthoxy, hydroxy, hydroxyméthyle, hydroxyéthyle, chloro, iodo, bromo, fluoro, trifluorométhyle, trifluorométhoxy, méthylsulfonyle, phényle ou cyano, ou bien R représente naphtyle, et tous les autres symboles ont les significations données dans la revendication 1.

4. Composés de formule générale (IV) 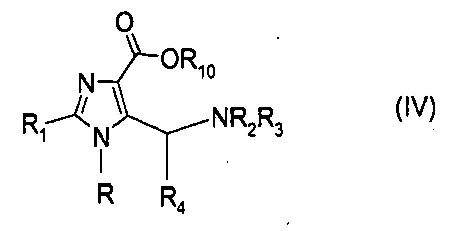 où R, R₁, R₂, R₃ et R₄ ont les significations données dans la revendication 1 et R₁₀ représente un atome d'hydrogène, un groupe C₁₋₄ alkyle linéaire ou ramifié ou un groupe benzyle, de tels composés étant utiles dans la synthèse de composés de formule générale (I).

5. Composés de formule générale (VII) 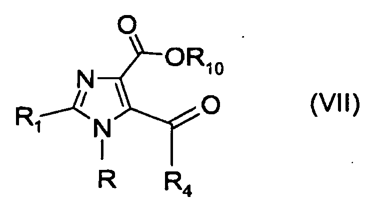 où R₁ a les significations données dans la revendication 1, R₄ représente un groupe méthyle, éthyle, propyle, isopropyle ou n-butyle et R représente un groupe C₁₋₈ alkyle ramifié ou linéaire, un groupe C₃₋₇- cycloalkyle, un groupe C₃₋₇-cycloalkyl-C₁₋₂-alkyle, un groupe C₃₋₈-hétéroalkyle ramifié ou linéaire, un groupe C₅₋₇-hétérocycloalkyle ou un groupe C₅₋₇-hétérocycloalkyl-C₁₋₂-alkyle, lesquels groupes peuvent être substitués avec un atome de fluor ou un groupe CF₃ ou OH, et R₁₀ représente un groupe méthyle, n-propyle, isopropyle, n-butyle, 2-butyle, isobutyle ou 2-méthyl-n-propyle ou un groupe benzyle.

6. Compositions pharmaceutiques contenant au moins un composé selon l'une quelconque des revendications 1-3 comme composant actif.

7. Composé selon l'une quelconque des revendications 1-3, ou sel de celui-ci, destiné à être utilisé comme médicament.

8. Utilisation d'un composé selon l'une quelconque des revendications 1-3 pour la préparation d'une composition pharmaceutique pour le traitement des troubles impliquant la neurotransmission avec des cannabinoïdes.

9. Utilisation selon la revendication 8 **caractérisée en ce que** lesdits troubles sont la psychose, l'anxiété, la dépression, les déficits de l'attention, les troubles de la mémoire, les troubles cognitifs, les troubles de l'appétit, l'obésité, en particulier l'obésité juvénile et l'obésité induite par des drogues, l'addiction, l'appétence, la dépendance aux drogues et les troubles neurologiques comme les troubles neurodégénératifs, la démence, la dystonie, la spasticité musculaire, les tremblements, l'épilepsie, la sclérose multiple, les lésions traumatiques du cerveau, l'attaque, la maladie de Parkinson, la maladie d'Alzheimer, l'épilepsie, la maladie de Huntington, le syndrome de Tourette, l'ischémie cérébrale, l'apoplexie cérébrale, le traumatisme craniocérébral, l'attaque, les lésions de la moelle épinière, les troubles neuro-inflammatoires, la sclérose en plaques, l'encéphalite virale, les troubles liés à la démyélinisation, ainsi que pour le traitement des troubles douloureux incluant les troubles douloureux neuropathiques et d'autres maladies impliquant la transmission avec des cannabinoïdes incluant le traitement du choc septique, du glaucome, du cancer, du diabète, du vomissement, de la nausée, de l'asthme, des maladies respiratoires, des troubles gastro-intestinaux, des troubles sexuels, de la cirrhose du foie, des ulcères gastriques, de la diarrhée et des troubles cardiovasculaires.

10. Utilisation selon la revendication 8 **caractérisée en ce que** lesdits troubles sont des troubles de l'alimentation, en particulier l'obésité, l'obésité juvénile et l'obésité induite par des drogues.

11. Utilisation d'un composé selon la revendication 1 pour la préparation d'une composition pharmaceutique pour le traitement des troubles de l'alimentation, en particulier de l'obésité, de l'obésité juvénile et de l'obésité induite par des drogues, **caractérisée en ce que** ladite composition pharmaceutique contient aussi au moins un inhibiteur de lipase.

12. Utilisation selon la revendication 11 **caractérisée en ce que** ledit inhibiteur de lipase est l'orlistat ou la lipstatine.
